# EUROPEAN PATENT APPLICATION

(11) **EP 3 690 020 A1**
(43) Date of publication of application: **05.08.2020**
(21) Application number: 18911017.4
(22) Date of filing: 19.03.2018
(51) Int. Cl.: C12M 3/00, C12N 5/071

(54) **PRODUCTION DEVICE, PRODUCTION SYSTEM AND PRODUCTION METHOD FOR CELL STRUCTURE**

(71) Applicant: Tissuebynet Corporation, Kita-ku Tokyo 115-0041 (JP)
(72) Inventor: ONO Jiro, Tokyo 115-0041 (JP)
(74) Representative: Forrest, Stuart
(86) International application number: PCT/JP2018/010795
(87) International publication number: WO 2019/180776

(57) **Abstract**

Provided are: a production device by which a cell structure having a three-dimensional structure is produced using a plurality of linear members; a production system therefor; and a production method therefor. The production device 100 comprises a top plate 110, pins 120A to 120D, a first slide plate 130, a second slide plate 140, a stopper 150, a base plate 160, an outer peripheral needle-shaped member 170 and an inner peripheral needle-shaped member 180. Cell aggregates 400 are put into a three-dimensional tubular space S1 that is defined by the outer peripheral needle-shaped member 170 and the inner peripheral needle-shaped member 180. Then, the top plate 110 is pressed downward on the accumulated cell aggregates 400. Thus, the cell aggregates 400 are immersed in a culture solution 210 and stuck together so that a tubular cell structure 500 is produced using the three-dimensional space S1 as a mold.

## Description

### Field

The present invention relates to the production of a three-dimensional cell structure or a three-dimensional cell construct using cell aggregates, and particularly relates to an apparatus method for a three-dimensional cell structure using strip-shaped and linear members formed vertically.

### Background

Requests, demand, and need for medical practice have only increased following globally increasing populations and life spans, and in recent years, regenerative medicine using cells is gathering attention as a new technique. Medical techniques of injecting individual cells into the body as is are already being implemented in other fields. While operation of these techniques is simple, there is a problem of the injected cells failing to become established at the desired location.

To this end, methods for fusing large numbers of cells and producing three-dimensional structures have been developed. The methods spatially hold cells in any shape in a petri dish, a gel support, a needle-shaped support, or the like and create three-dimensional structures. There are mainly two applications of these methods.

The first is the manufacture of artificial tissues and organs for transplanting into human bodies. Artificial tissues are made into a form wherein a portion thereof expresses functions, and transplanting into human bodies is the final goal. However, suitable evaluation and certification is necessary before transplanting into human bodies, and long-term efforts are necessary. Currently, directly producing organs having complicated shapes is difficult, and production is limited to organs having simple shapes (blood vessels and the like).

The second is methods for utilizing these three-dimensional cell structures as specimens in toxicity testing, drug effect determination, pathologic determination, embryology, and the like. By creating a three-dimensional cell structure using only human cells, reproducing the internal environment or making an environment similar thereto, and carrying out the tests above, experiments imitating the internal environment become possible outside the body. Thereby, efficient drug discovery research, personalized diagnosis and medication, and observational studies of organogenesis for various organs, and the like become possible. Particularly, in administration of medication for cancer, prediction and determination of the period and effect thereof are difficult, but by, for example, using the present art to first evaluate and certify medication administration trials outside the body using samples created from cancer tissues of patients using such, it is expected that these tests will become indicators for ascertaining medicine effects.

There are two types of cells, suspension cells and scaffold-dependent adherent cells. Blood system and immune system cells fall under the former, and organ, skin, and bone cells and the like fall under the latter. Adherent cells cannot survive for a long period in a suspended state in a solution, and adherence to a scaffold such as a glass petri dish or a hydrogel is necessary for survival and propagation. When adherent cells are placed in a nonadherent environment, the cells seek a scaffold, adhere to each other, and form cell aggregates, and when fellow cell aggregates are further placed in an environment where they mutually adhere by some method, they adhere, fuse and form even larger three-dimensional cell structures. This phenomenon is widely known, and nonpatent literature 1 to 6 teach concrete examples thereof. As in nonpatent literature 1, the phenomenon of cell aggregates (also expressed as cluster in the present document) fusing has been long known since the 1960s. In particular, nonpatent literature 6 teaches the idea of treating three-dimensional cell structures as "building blocks (building block)" and suggests that various cells can be used.

A cell cluster (spheroid) is a substantially round aggregate consisting only of cells, and a cell aggregate aggregate (cell aggregate) indicates an aggregate configured with cell clusters, cells, and other substances.

Patent literature 1 discloses a structure plug production method capable of creating a structure of any shape from only cells without the use of a support. Specifically, cell aggregates are placed in a chamber having micropores through which culture solution can pass in only a base, an amount of culture solution is contained in the chamber in an amount such that a portion of the cell aggregates contact a gaseous phase, and the cell aggregates are cultured in a quantity of culture solution in excess relative to the culture solution in the chamber.

Furthermore, a dispensing method taught in patent literature 2 wherein cell aggregates are dispensed from a nozzle of a bioprinter onto a level surface and a pin frog method taught in patent literature 3 wherein cell clusters are passed through needle-shaped supports are known as methods for producing three-dimensional cell structures. Moreover, patent literature 4 discloses a method wherein cultured cells plate-cultured on a permeable sheet are stacked, including the sheet, on other plate-cultured cultured cells, and three-dimensional cells are produced.

### Prior Art Literature

### Patent Literature

Patent Literature 1: Patent No. 4122280
Patent Literature 2: US Patent No. 8852932
Patent Literature 3: Patent No. 4517125 (International Application No. PCT/JP2008/056826)
Patent Literature 4: WO 2005/047496

Non Patent Literature 1: PLOS ONE, Journal. Pone. 0136681, "Scaffold-Free Tubular Tissues Created by a Bio-3D printer Undergo Remolding and Endothelialization when Implanted in Rat Aortae", Manabu Itoh et al, September 1, 2015
Non Patent Literature 2: Gordon R, Goel NS, Steinberg MS, Wiseman LL. A rheological mechanism sufficient to explain the kinetics of cell sorting. J Theor Biol. 1972; 37:43-73. [PubMed: 4652421]
Non Patent Literature 3: Jakab K, Damon B, Marga F, Doaga O, Mironov V, Kosztin I, Markwald R, Forgacs G. Relating cell and tissue mechanics: implications and applications. Dev. Dyn. 2008; 237:2438-2449. [PubMed: 18729216]
Non Patent Literature 4: Jakab K, Neagu A, Mironov V, Markwald RR, Forgacs G. Engineering biological structures of prescribed shape using self-assembling multicellular systems. Proc Natl Acad Sci USA. 2004; 101:2864-2869. [PubMed: 14981244]
Non Patent Literature 5: Perez-Pomares JM, Foty RA. Tissue fusion and cell sorting in embryonic development and disease: biomedical implications. Bioessays. 2006; 28:809-821. [PubMed: 16927301]
Non Patent Literature 6: Organ printing: Tissue spheroids as building blocks" Biomaterials. Vladimir Mironov, Richard P. Visconti, Vladimir Kasynocv, Gabor Forgacs, Christopher J. Drake, and Roger R. Markwald, 2009 April; 30(12):2164-2174. doi:10.1016

### Summary of Invention

### Problem to be Solved by Invention

Most of the dispensing methods taught in patent literature 2 are methods wherein a mixture called a bio-ink configured with cell clusters and a binder such as bio-gel or collagen is discharged onto a level surface or three-dimensional cell structure production methods wherein a material capable of retaining its shape that hardens easily such as a bio-gel or collagen is first made into a scaffold (scaffold) into which cell clusters are injected, but there is the disadvantage that intercellular contact is disturbed. Furthermore, since the shape of three-dimensional cell structure produced by this method depends on the shape-retaining ability of the binder, restrictions are imposed upon the size and shape (especially on the height) of the three-dimensional cell structure. Moreover, the binder remains inside the three-dimensional cell structure, so a problem remains that a harmful effect on the cells given by the binder cannot be eliminated, and an additional confirmation of the evaluation is necessary when transplanting into human bodies or when measuring effects. The pin frog method of patent literature 3 also restricts the shape due to the needle-shaped support.

An object of the present invention is to provide a to solve such conventional problems and provide a production device, a production system, and a production method for producing a three-dimensionally structured cell structure using a plurality of linear members.

### Means for Solving Problem

The cell structure production device of the present invention is a production device for a cell structure, the production device having at least one upper member, at least one lower member, and a plurality of linear members disposed between the upper member and the lower member, wherein one end of each of the linear members is supported by the upper member, and the other end is supported by the lower member, and the plurality of linear members define a three-dimensional space, and the space is capable of accommodating a plurality of cell aggregates.

In one embodiment, the plurality of linear members includes a first linear member substantially defining a first surface, and a second linear member substantially defining a second surface separated from the first surface, and the production device accommodates a cell aggregate in a space formed by the first surface and the second surface. In one embodiment, the first surface defines an outer shape of the cell structure, and the second surface defines the inner shape of the cell structure. In one embodiment, the first surface and the second surface are circular. In one embodiment, a cell aggregate accommodated within the space is exposed to a liquid such as a culture medium through the plurality of linear members. In one embodiment, the upper member includes a substantially flat member, and an input hole for inputting the cell aggregate within the space is formed on the flat member. In one embodiment, the production device further includes a slide member having a plurality of a through hole formed between the upper member and the lower member, the plurality of linear members penetrating the plurality of a through hole, wherein the slide member is capable of moving between the upper member and the lower member. In one embodiment, the production device further includes a plurality of a strut for connecting the upper member and the lower member, wherein the slide member is guided by the plurality of a strut and is capable of moving in a direction close to or separated from the upper member. In one embodiment, at least one of the plurality of a strut is provided with a control member for controlling movement of the slide member. In one embodiment, the first and second linear members define a structure of blood vessels. In one embodiment, the first and second linear members define a structure of a valve of a heart. In one embodiment, the plurality of linear members is a member produced by a three-dimensional printer. In one embodiment, the plurality of linear members is a member produced by a wire-bonding method, such as that used in semi-conductor production. In one embodiment, the plurality of linear members is a member produced by formation using a mold.

The production system of the present invention includes the production device, a container capable of accommodating the production device and for accommodating a culture medium for providing nourishment to a cell aggregate in the production device, and a pump for circulating the culture medium. In one embodiment, the production system includes a shaking means for shaking the container. In one embodiment, the production system includes means for arbitrarily supplying a culture medium to a defined site.

The production method for a cell structure of the present invention is a production method for cell structure utilizing the production device, having a step of supplying a plurality of cell aggregates to the space, a step of culturing the cell aggregates and fusing the cell aggregates, and a step of disengaging the plurality of linear members from the fused cell aggregates. In one embodiment, the slide member is moved to disengage the plurality of linear members from cell aggregates. In one embodiment, the step of disengaging the plurality of linear members includes a step of separately disengaging each linear member.

### Effect of the Invention

According to the present invention, since a three-dimensional space is formed by a plurality of linear members supported by at least one upper member and at least one lower member, and the space is enabled to accommodate a plurality of cell aggregates, a cell aggregate of an arbitrary shape or structure can be easily produced by a plurality of linear members.

### Brief Description of Drawings

[FIG. 1] is a perspective diagram illustrating a cell structure production device according to an example of the present invention.
[FIG. 2] is an exploded perspective diagram illustrating a cell structure production device according to an example of the present invention.
[FIG. 3] is a diagram describing a top plate according to an example of the present invention.
[FIG. 4] is a diagram describing a pin according to an example of the present invention.
[FIG. 5] is a diagram describing a first slide plate according to an example of the present invention.
[FIG. 6] is a diagram describing a second slide plate according to an example of the present invention.
[FIG. 7] is a diagram describing a stopper according to an example of the present invention.
[FIG. 8] is a diagram describing a base plate according to an example of the present invention.
[FIG. 9] is a diagram describing a needle-shaped member according to an example of the present invention.
[FIG. 10] is a diagram describing a tubular three-dimensional space according to an example of the present invention.
[FIG. 11] is a flow chart showing a cell structure production method according to an example of the present invention.
[FIG. 12] is a diagram describing inputting cell aggregates according to an example of the present invention.
[FIG. 13] is a diagram describing lowering a top plate according to an example of the present invention.
[FIG. 14A] is a diagram illustrating an example of culturing cell aggregates.
[FIG. 14B] is a diagram illustrating an example of culturing using a plurality of a nozzle.
[FIG. 14C] is a diagram illustrating an example of culturing, having a plurality of a nozzle and simultaneously supplying culture medium of separate systems.
[FIG. 15A] is a diagram describing taking out a produced cell structure.
[FIG. 15B] is a diagram describing taking out a produced cell structure.
[FIG. 16] is a perspective diagram illustrating a cell structure production device according to another example of the present invention.
[FIG. 17] is an exploded perspective diagram illustrating a cell structure production device according to another example of the present invention.
[FIG. 18] is a diagram exemplifying a shape of a cell structure according to another example of the present invention.
[FIG. 19A] is a diagram describing molding a cell structure according to another example of the present invention.
[FIG. 19B] is a schematic cross-sectional diagram describing molding a cell structure according to another example of the present invention.
[FIG. 20A] is a diagram illustrating an example of culturing cell aggregates according to another example of the present invention.
[FIG. 20B] is a diagram illustrating an example of culturing cell aggregates using a plurality of a nozzle according to another example of the present invention.
[FIG. 20C] is a diagram illustrating an example of culturing, having a plurality of a nozzle and simultaneously supplying culture medium of separate systems, according to another example of the present invention.
[FIG. 21] is a flow chart showing a cell structure production method according to another example of the present invention.

### Embodiments of Invention

A device for producing a three-dimensional cell construct according to an embodiment of the present invention defines the three-dimensional structure of a cell aggregate by a plurality of vertically disposed linear or needle-shaped members and produces a three-dimensional cell structure according to the three-dimensional structure. The linear or needle-shaped members can be processed into any shape and can be configured from steel material such as stainless steel, plastic, biodegradable material, or other soft materials such that they are easy to pull out from the cell structure. In one embodiment, a plurality of cell aggregates supplied to the production device is cultured using a solution containing nutrients and fused into one cell structure. Furthermore, the linear or needle-shaped members can be made in any shape using a three-dimensional printer. The three-dimensional printer generates members defining any three-dimensional structure based on three-dimensional data. Furthermore, it is also possible to mold the linear or needle-shaped members using a mold having a desired shape. Moreover, it is also possible to make such using a wire bonder method used for wiring during semiconductor production. Note, the scale of the drawings is exaggerated for understanding the present invention and is not necessarily the same as the size of an actual product or the like.

### Examples

Next, examples of the present invention will be described with reference to the drawings. The present example describes an example for producing a tubular (tubular) cell structure using a three-dimensional cell construct production device. The tubular cell structure is, for example, a blood vessel.

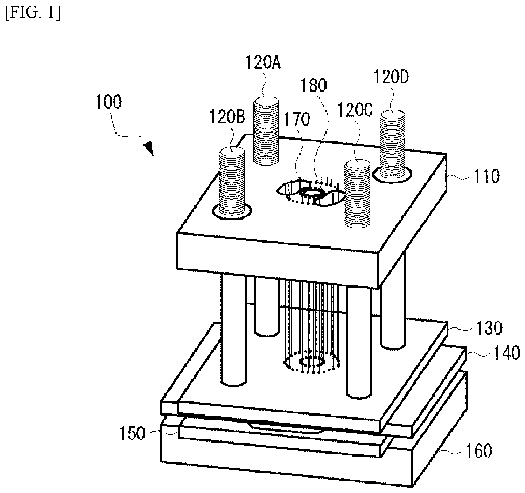

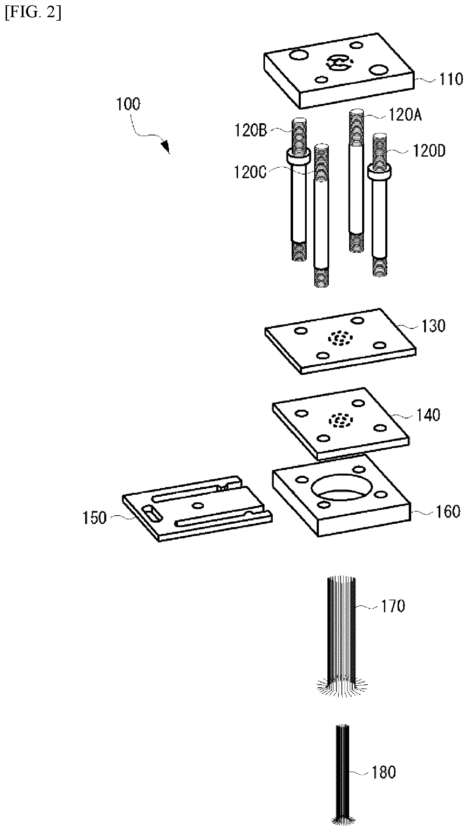

FIG. 1 is a perspective diagram illustrating a cell structure production device according to an example of the present invention, and FIG. 2 is an exploded perspective diagram illustrating a cell structure production device according to an example of the present invention. a cell structure production device 100 of the present example is configured including a top plate 110, four pins 120A to 120D (hereinafter, may be collectively referred to as pins 120), a substantially rectangular first slide plate 130, a substantially rectangular second slide plate 140, a stopper 150, a base plate 160, outer circumference needle-shaped members 170, and inner circumference needle-shaped members 180.

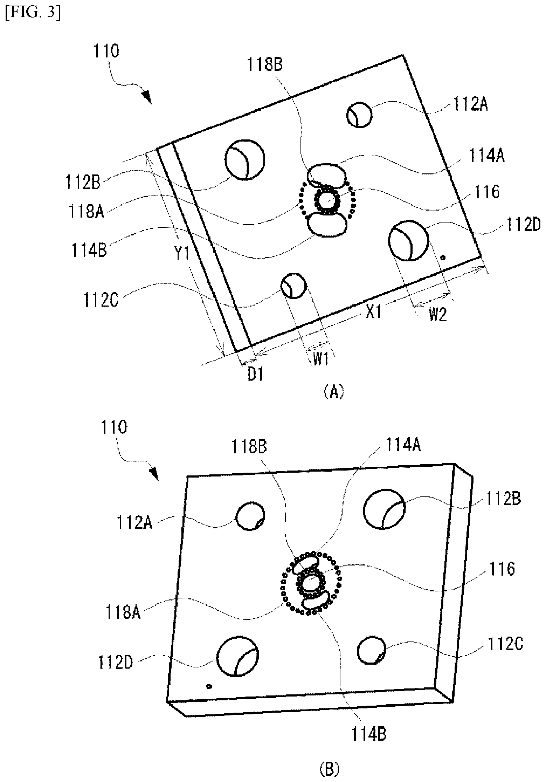

FIG. 3 is a perspective diagram of the top plate, FIG. 3(A) is a diagram from above the top plate 110, and FIG. 3(B) is a diagram from below the top plate 110. The top plate 110 is configured from a substantially rectangular, flat plate-shaped member having lengths X1 and Y1 and a thickness D1. Substantially circular through holes 112A, 112B, 112C, and 112D (hereinafter, may be collectively referred to as through holes 112) are formed in the top plate 110 near four corner portions. A diameter W1 of one diagonal pair of the through holes 112A and 112C is smaller than a diameter W2 of another diagonal pair of the through holes 112B and 112D.

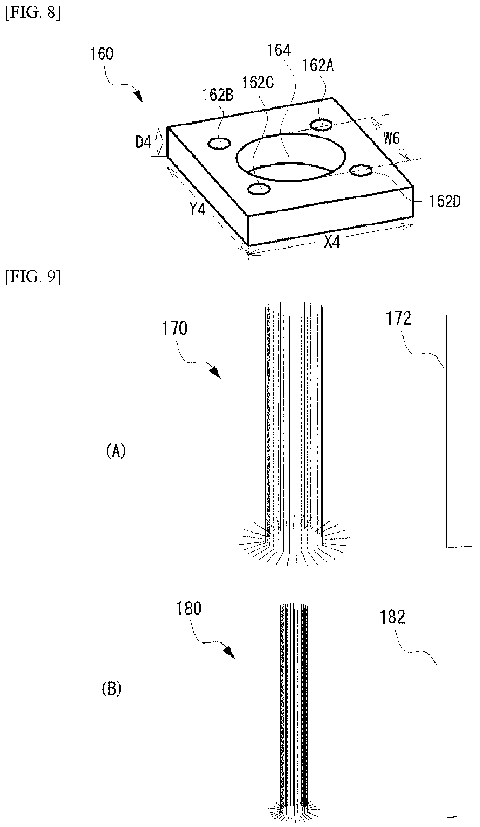

A pair of input holes 114A and 114B (hereinafter, may be collectively referred to as deposit holes 114) in a substantially elliptical shape are formed near a center of the top plate 110. The deposit holes 114 penetrate the top plate 110 and preferably have a bowl shape in which a diameter on a front side is larger than a diameter on a back side. A culture medium circulation hole 116 is formed between the pair of input holes 114A and 114B. The culture medium circulation hole 116 is a substantially circular through hole and is positioned so as to be sandwiched by the input holes 114. A plurality of needle holes 118A for inserting and supporting the outer circumference needle-shaped members 170 and a plurality of needle holes 118B for inserting and supporting the inner circumference needle-shaped members 180 as illustrated in FIG. 9 are further formed in the top plate 110. The needle holes 118A and 118B are through holes disposed in circles substantially concentric with the culture medium circulation hole 116.

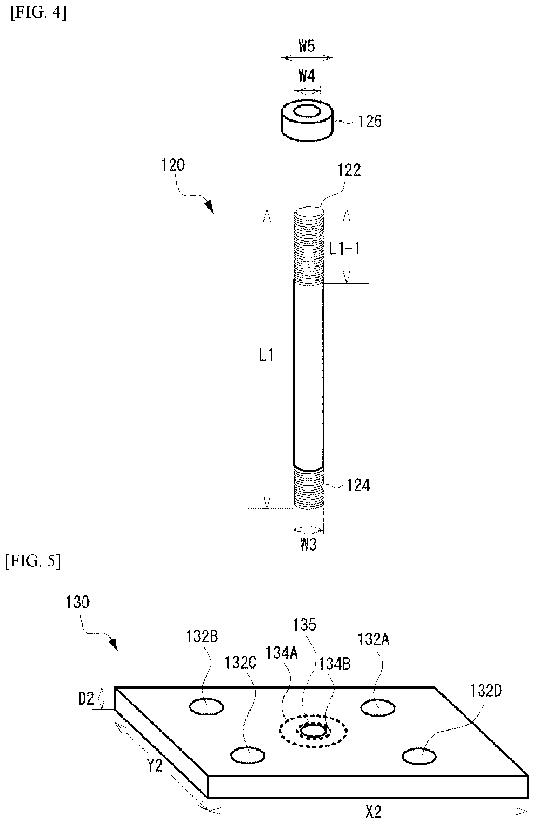

FIG. 4 exemplifies one pin. A pin 120 is a long, narrow columnar member having a length L1 and a diameter W3 and is configured of, for example, a steel material. The diameter W3 is substantially the same diameter as or slightly smaller than the diameter W1 of the through holes 112A and 112C, and the pin 120 can pass through the through holes 112. Male screw parts 122 and 124 are formed on both ends of the pin 120, and a ring member 126 engaging with the male screw part 122 is attached to the male screw part 122. The ring member 126 has an inner diameter W4 substantially equal to the diameter W3 of the pin 120, and the position thereof can be changed by rotating. The ring member 126 can move within a range of an axial length L1-1 of the male screw part 122. Note, the ring member 126 may be fixed to the pin 120.

A outer diameter W5 of the ring member 126 is substantially the same diameter as or slightly smaller than the diameter W2 of the through holes 112B and 112D, and the ring member 126 can pass through the through holes 112B and 112D. As illustrated in FIG. 2, the ring member 126 is mounted on the pins 120B and 120D and is not mounted on the pins 120A and 120C. The male screw part 124 is attached to the base plate 160 described later.

FIG. 5 illustrates the first slide plate. The first slide plate 130 is a substantially rectangular flat plate-shaped member having lengths X2 and Y2 and a thickness D2. The first slide plate 130 has four through holes 132A, 132B, 132C, and 132D (hereinafter, may be collectively referred to as through holes 132) of the same size formed at positions matching the through holes 112A, 112B, 112C, and 112D of the top plate 110, and the pins 120A, 120B, 120C, and 120D are inserted into these through holes. The first slide plate 130 can move in the axial direction of the pins 120 by using the pins 120 inserted into the through holes 132 as a guide. Furthermore, a plurality of needle holes 134A and 134B (hereinafter, may be collectively referred to as needle holes 134) into which the outer circumference needle-shaped members 170 and the inner circumference needle-shaped members 180 can be inserted are formed near the center of the first slide plate 130 at positions matching the needle holes 118A and 118B. Additionally, a culture medium circulation hole 135, which is a substantially circular through hole, is formed inside the needle hole 134B.

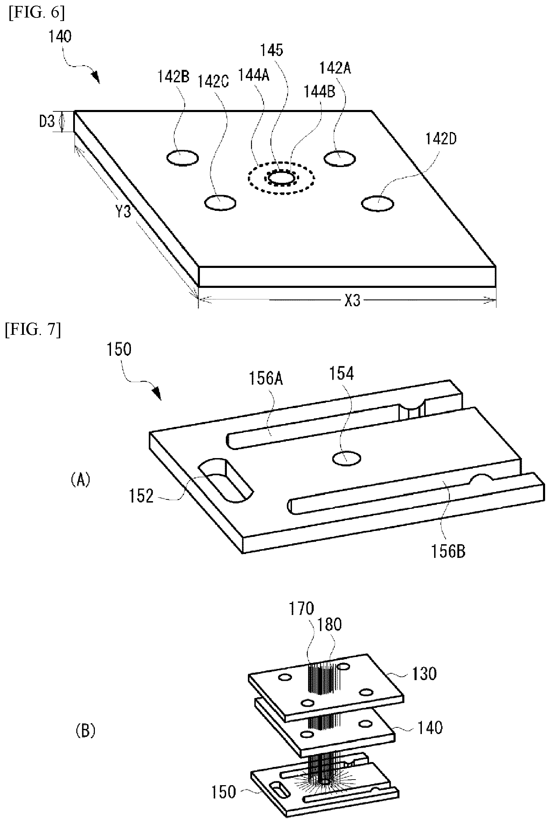

FIG. 6 illustrates the second slide plate. The second slide plate 140 is a substantially rectangular plate-shaped member having lengths X3 and Y3 and a thickness D3. In one example, its relationship to the first slide plate 130 is X3<X2, Y3>Y2. The second slide plate 140 has through holes 142A, 142B, 142C, and 142D (hereinafter, may be collectively referred to as through holes 142) of the same size formed at positions matching the four through holes 132A, 132B, 132C, and 132D of the first slide plate 130, and the pins 120A, 120B, 120C, and 120D are inserted into these through holes 142, respectively. Furthermore, similarly to the first slide plate 130, a plurality of needle holes 144A and 144B (hereinafter, may be collectively referred to as needle holes 144)into which and by which the outer circumference needle-shaped members 170 and the inner circumference needle-shaped members 180 can be inserted and supported are formed near the center of the second slide plate 140 at positions matching the needle holes 118A and 118B. Additionally, a culture medium circulation hole 145, which is a substantially circular through hole, is formed inside the needle hole 144B.

FIG. 7 illustrates the stopper. As illustrated in FIG. 7A, the stopper 150 includes a grip part 152, a culture medium circulation hole 154, and slide guides 156A and 156B (hereinafter, may be collectively referred to as slide guides 156) capable of passing through the pins 120. The grip part 152 slightly protrudes from between the second slide plate 140 and the base plate 160 so as to be gripped by the production device 100 illustrated in FIG. 1, and by pulling the grip part 152, the stopper 150 can be pulled out of the production device 100. The culture medium circulation hole 154 is a through hole for supplying a culture solution to the cell structure. The slide guides 156 are long, narrow guides, and one end of the long, narrow guides is open. Accordingly, via these openings, the pins 120A and 120B can be passed through the slide guide 156A, and the pins 120C and 120D can be passed through the slide guide 156B. By having such a structure, the stopper 150 can be inserted and removed between the second slide plate 140 and the base plate 160 even when, for example, the production device 100 such as illustrated in FIG. 1 is in an assembled state.

FIG. 7(B) is a diagram illustrating a relationship between the stopper 150 and other members. The outer circumference needle-shaped members 170 and the inner circumference needle-shaped members 180 are disposed on the stopper 150. Preferably, they are disposed such that the culture medium circulation hole 154 enters inside the inner circumference needle-shaped members 180. The outer circumference needle-shaped members 170 and the inner circumference needle-shaped members 180 pass through the first slide plate 130 and the second slide plate 140 via the needle holes 134 and 144. Accordingly, the outer circumference needle-shaped members 170 and the inner circumference needle-shaped members 180 are fixed by the stopper 150 and the second slide plate 140, and the needle-shaped members are fixed even when the first slide plate 130 is moved.

FIG. 8 illustrates the base. The base 160 is a substantially rectangular member having lengths X4 and Y4 and a thickness D4. The base 160 is provided with female screw parts 162A, 162B, 162C, and 162D (hereinafter, may be collectively referred to as female screw parts 162) of substantially the same diameter, corresponding to the male screw parts 124 of the pins 120A, 120B, 120C, and 120D, respectively. The pins 120 are screw-fixed to the base plate 160 by the female screw parts 162 and held vertically. Furthermore, a through hole 164 having a diameter W6 is provided in a center of the base plate 160, and when the stopper 150 is in a pulled out state, the outer circumference needle-shaped members 170 and the inner circumference needle-shaped members 180 can be pulled out of the through hole 164.

FIG. 9 illustrates the needle-shaped members. The outer circumference needle-shaped members 170 have a plurality of needle-shaped members 172 disposed in a circular shape, one end of the needle-shaped members 172 is bent at a substantially right angle, and the other end can pass through the needle holes 144A, 134A, and 118A. The inner circumference needle-shaped members 180 have a plurality of needle-shaped members 182 disposed in a circular shape, one end of the needle-shaped members 182 is bent at a substantially right angle, and the other end can pass through the needle holes 144B, 134B, and 118B. These needle-shaped members form a tubular three-dimensional space between the top plate 110 and the first slide plate 130. The needle-shaped members are configured from, for example, a stainless steel material, a plastic material, a biodegradable material, or the like. The cross-sectional shape of the needle-shaped member is arbitrary, but desirably, it is, for example, circular or elliptical in order to enable easy detachment from the cell aggregate, and has a uniform thickness. However, the cross-sectional shape may be triangular or prismatic. Furthermore, the surface of the needle-shaped members may be coated with a non-adhesive material for enabling easy detachment from the cell aggregate.

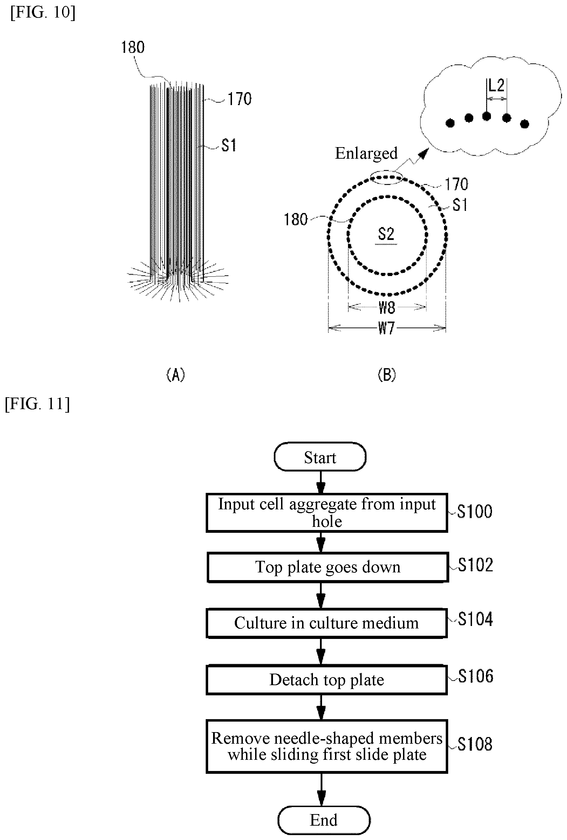

FIG. 10 is a diagram describing a tubular three-dimensional space according to an example of the present invention. FIG. 10(A) is a diagram wherein the outer circumference needle-shaped members 170 and the inner circumference needle-shaped members 180 of the production device are taken out. The inner circumference needle-shaped members 180 are inserted into the inner space of the outer circumference needle-shaped members 170, and the inner circumference needle-shaped members 180 and the outer circumference needle-shaped members 170 form a tubular three-dimensional space S1. FIG. 10(B) is a bird's-eye diagram of FIG. 10(A) (the bent portion of the needle-shaped members is omitted). A tubular cell structure is produced by supplying the cell aggregate into the three-dimensional space S1 by a cell structure production method described later. Accordingly, an outer diameter of the tubular cell structure is defined by a diameter W7 of the outer circumference needle-shaped members 170, and an inner diameter is defined by a diameter W8 of the inner circumference needle-shaped members 180. A space S2 is a space of the tubular cell structure. Note, in FIG. 10(B), a portion of the outer peripheral needle-shaped members 170 is enlarged, and an interval L2 between the needle-shaped members is smaller than the supplied cell aggregate. That is, the interval L2 is an interval such that the cell aggregate does not leak. The same is true for an interval between the needle-shaped members of the inner circumference needle-shaped members 180. The interval L2 may be an interval not allowing the cell aggregate to leak out, and the intervals between all the needle-shaped members need not necessarily be constant. Note, the interval L2 between the needle-shaped members can allow a culture solution 210 described later to pass through, and thus it is possible to supply a culture medium containing nutrients from between the needle-shaped members.

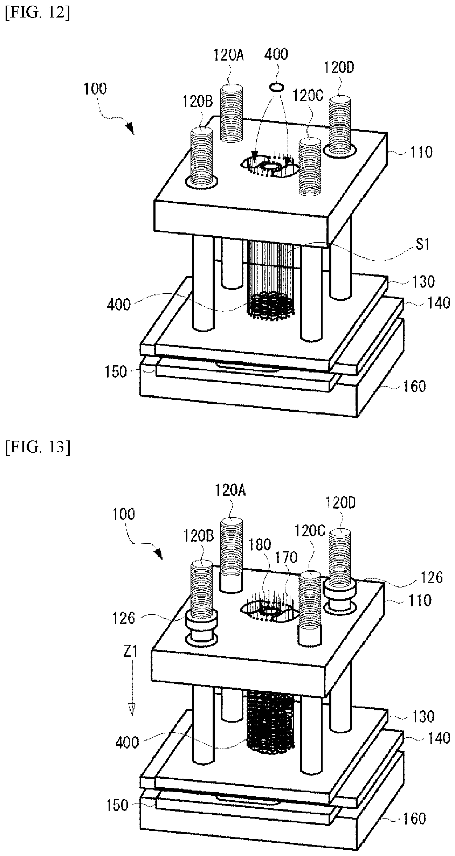

Next, a method for producing a cell structure using the production device 100 according to the present example will be described. FIG. 11 is a flow chart showing a cell structure production method according to an example of the present invention. In the cell structure production method, first, the cell aggregate (spheroid) is supplied from the input holes 114 of the production device 100 (S100). FIG. 12 is a diagram describing inputting the cell aggregates according to an example of the present invention. When cell aggregates 400 larger than the interval L2 between the needle-shaped members are charged from the input holes 114, cell aggregates 400 accumulate in the tubular three-dimensional space S1. Next, once the cell aggregates 400 have accumulated to an extent, as illustrated in FIG. 13, the top plate 110 is lowered in the Z1 direction such that the cell aggregates 400 adhere to each other and do not move around (S102).

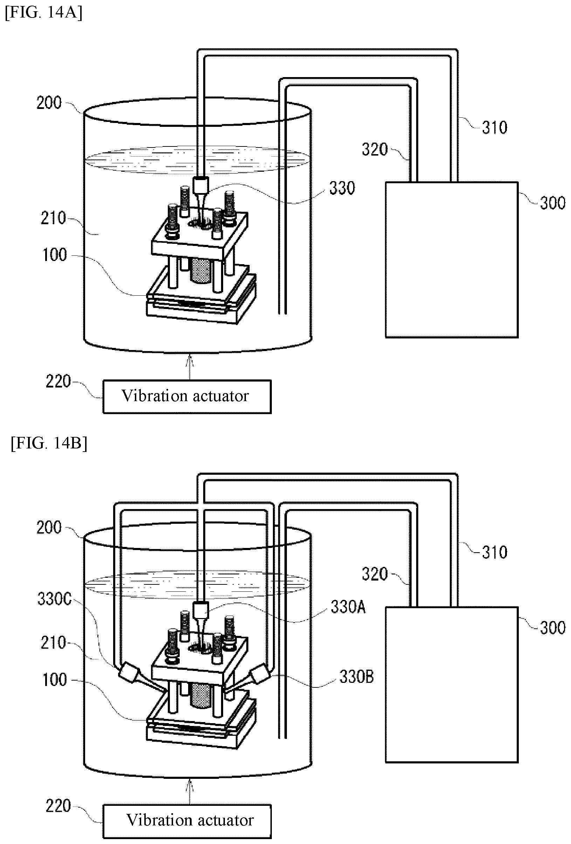

Next, the cell aggregates made to accumulate in a tubular shape are cultured (S104). FIG. 14A is a diagram illustrating an example of culturing the cell aggregates. The accumulated cell aggregates 400 are put into a container 200 along with the whole production device 100. The container 200 contains the culture solution 210 into which nutrients for culturing the cell aggregates 400 are dissolved and supplies the nutrients to the cell aggregates 400. Preferably, a circulation pump 300 is provided, the culture solution is sucked up by a tube 320, and the culture solution 210 is circulated by discharging the culture solution from a tube 310. A nozzle 330 is connected to a tip of the tube 310, and by circulating the culture solution through the culture medium circulation holes 116, 135, and 145, the culture solution can be spread over the entirety of the cell aggregates. More preferably, a vibration actuator 220 for shaking the container 200 may be provided, and the culture may be performed while shaking the container 200. Note, when culturing the cell aggregates 400, it is preferable to control the production system illustrated in FIG. 14A by temperature, humidity, light amount, and the like suitable for culturing.

FIG. 14B is a diagram illustrating an example of culturing using a plurality of a nozzle. In this case, in addition to a nozzle 330A inserted into the medium circulation hole 116, a nozzle 330B and a nozzle 330C for supplying culture medium from around the cell aggregates 400 are provided. In the production system illustrated in this drawing, the culture solution sucked up from the tube 320 is again discharged from the three nozzles 330A, 330B, 330C via the tube 310. The circulation pump 300 be provided with a function of filtering the sucked-up culture medium. Furthermore, the nozzle 330B and the nozzle 330C may have a fixing mechanism capable of being disposed at any position and may efficiently supply the culture solution to any section of the cell aggregates 400. Moreover, the nozzle 330B and the nozzle 330C may be enabled to move on any trajectory or to any position by a driving device or driving mechanism such as an actuator.

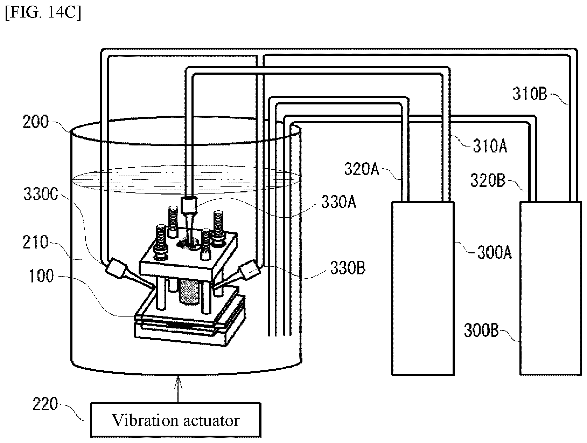

FIG. 14C is a diagram illustrating an example of culturing, having a plurality of a nozzle and simultaneously supplying culture medium of separate systems. The production system illustrated in this drawing has separate system circulation pumps 300A and 300B, and each pump is connected to discharge tubes 310A and 310B and suction tubes 320A and 320B, respectively. The culture medium sucked up from the tube 320A is discharged from the nozzle 330A via the tube 310A. Furthermore, the culture medium sucked up from the tube 320B is discharged from the nozzles 330B and 330C via the tube 310B. In this case, in addition to being able to supply the culture medium to any defined location of the cell aggregates 400, the nozzle 330B and the nozzle 330C are connected to tanks of different systems, and thus it is possible to activate only one of the circulation pumps. Furthermore, the nozzle 330B and the nozzle 330C may be enabled to move on any trajectory or to any position by a driving device or driving mechanism such as an actuator.

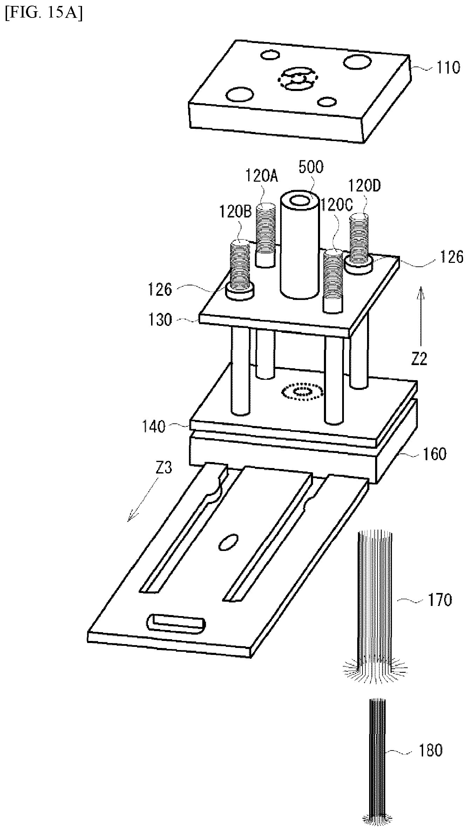

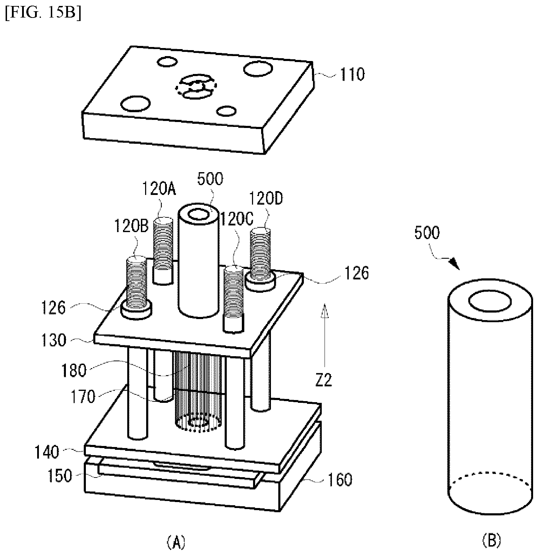

The cell aggregates 400 adhere to each other due to the culture in S104 and grow into a cell structure along the three-dimensional space S1. FIG. 15A and FIG. 15B are diagrams describing taking out a produced cell structure. As illustrated in FIG. 15A, when the cell aggregates 400 grow into a cell structure 500, first the top plate 110 is taken off (S106). Next, while sliding the first slide plate 130 in the Z2 direction, the cell structure 500 is pulled out from the outer circumference needle-shaped members 170 and the inner circumference needle-shaped members 180 (S108). At this time, when it is difficult to remove the cell structure 500 from the needle-shaped members, the stopper 150 can be pulled out in the Z3 direction, and one or more needle-shaped members can be taken out from the through hole 164. When the cell structure 500 is easily pulled out from the needle-shaped members, as illustrated in FIG. 15B(A), by sliding the first slide plate 130 without removing the stopper 150, the cell structure 500 can be removed. When pulled out as illustrated in FIG. 15B (A), the outer circumference needle-shaped members 170 and the inner circumference needle-shaped members 180 remain in an assembled state in the production device 100, thus saving the trouble of passing the needle-shaped members through the needle holes again. Thus, the tubular cell structure 500 is produced on the first slide plate 130. FIG. 15B (B) is a diagram exemplifying a produced cell structure.

To prevent the cell structure 500 from adhering to the needle-shaped members when taking the cell structure 500 out of the production device 100, it is preferable that the outer circumference needle-shaped members 170 and the inner circumference needle-shaped members 180 are configured from a material such as, for example, stainless steel, nylon, or polyester, and that their size is around 10 *µ*m in diameter. It is more preferable that the needle-shaped members are coated with a non-adhesive such as P-HEMA and disinfected with ethanol or the like. It is even more preferable that a non-adhesive is applied in advance to the contact surface between the top plate 110 and the cell structure 500, the contact surface between the first slide plate 130 and the cell structure 500, or the like, so that the cell structure 500 may be easily taken out.

In the foregoing example, two sets of the outer circumference needle-shaped members 170 and the inner circumference needle-shaped members 180 were used to define the contours of the outer circumference surface and the inner circumference surface of the blood vessel, respectively; however, this is one example. The contour and shape of a cell structure having a more complicated shape may be further defined by three sets or more of needle-shaped members. Further, the contour or contour surface defined by needle-shaped members is not limited to a spherical surface or a curved surface, and may be a straight line. For example, it is also possible to define the contour or shape of a prismatic cell structure, a polygonal cell structure, or the like.

Furthermore, in the foregoing example, the needle-shaped members are supported by directly inserting the end parts of the outer circumference needle-shaped members 170 and the inner circumference needle-shaped members 180 into the through holes of the top plate and the second slide plate; however, this is one example. For example, these may be indirectly attached to the top plate and the second slide plate via a dedicated support member or the like.

Next, another example of the present invention will be described with reference to drawings. In another example, an example wherein a production device of a three-dimensional cell construct is used to produce a cell structure having a valve part will be described. The cell structure having a valve part is, for example, a cell structure modeled after a heart valve.

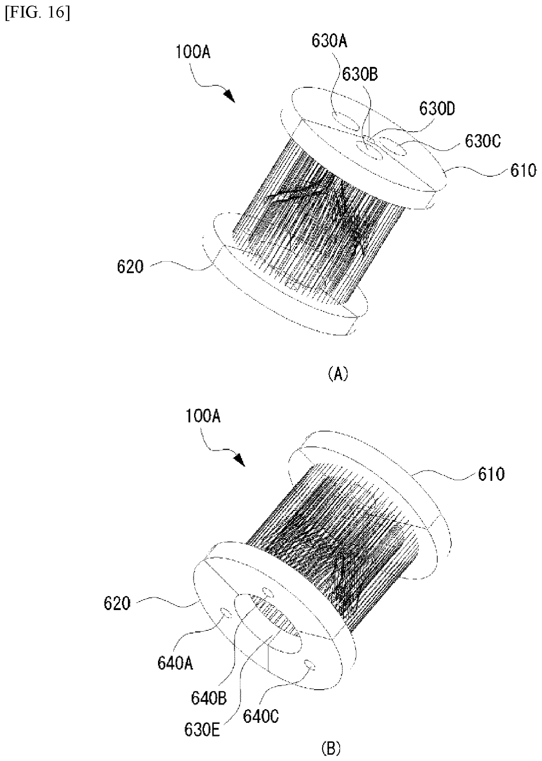

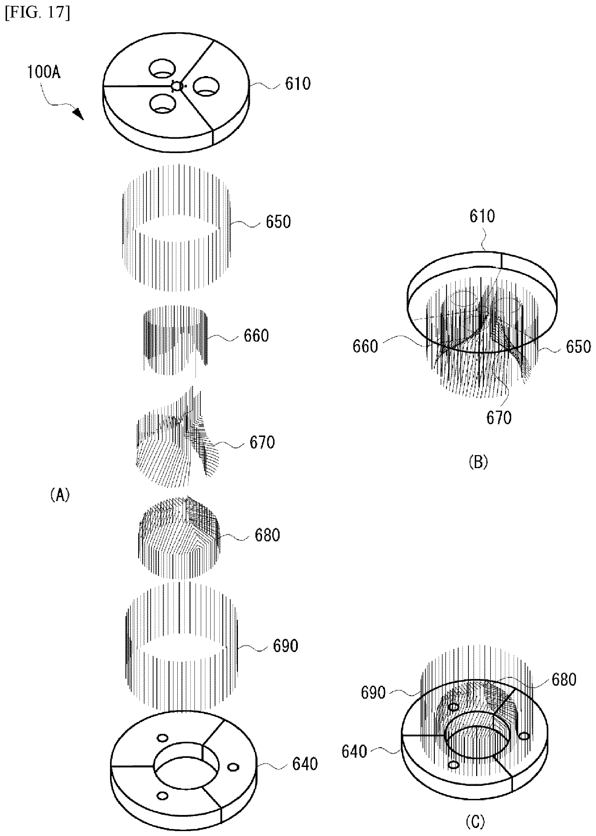

FIG. 16 is a perspective diagram illustrating a cell structure production device according to another example of the present invention., and FIG. 17 is a diagram illustrating an exploded perspective diagram of the cell structure production device according to another example. A production device 100A for a cell structure according to the present example includes an upper frame 610, a lower frame 620, a culture medium circulation hole 630A, 630B, 630C, 630D, and 630E (hereinafter collectively referred to as culture medium circulation hole 630) provided on the upper frame 610 and the lower frame 620, a input hole 640A, 640B, and 640C (hereinafter collectively referred to as input hole 640) provided on the lower frame 620, a first outer needle-shaped member 650, a valve-molded needle-shaped member upper part 660, a valve-molded needle-shaped member middle part 670, a valve-molded needle-shaped member lower part 680, and a second outer needle-shaped member 690. The upper frame 610 and the lower frame 620 are configured from three pieces, as illustrated in FIGS. 16 and 17. Since the upper frame 610 and the lower frame 620 are easily produced by a three-dimensional printer or the like, the upper frame 610 and the lower frame 620 are configured by joining each piece. However, it is not at all necessary to split into each piece; the upper frame 610 and the lower frame 620 may each be configured from one member.

The first outer needle-shaped member 650, the valve-molded needle-shaped member upper part 660, and the valve-molded needle-shaped member middle part 670 are fixed to a surface of the upper frame 610 (surface opposing the lower frame 620), as illustrated in FIG. 17 (B). Fixing is performed in the same way as the first example, wherein a plurality of a through hole is formed on the upper frame 610, and the needle-shaped members are inserted therein. The valve-molded needle-shaped member lower part 680 and the second outer needle-shaped member 690 are fixed to a surface of the lower frame 620 (surface opposing the upper frame 610) by the same method, as illustrated in FIG. 17 (C). In the production device 100A of a cell structure according to the present example, such needle-shaped members define a contour of a three-dimensional structure, thereby enabling production of a cell structure of a heart valve having a complex shape.

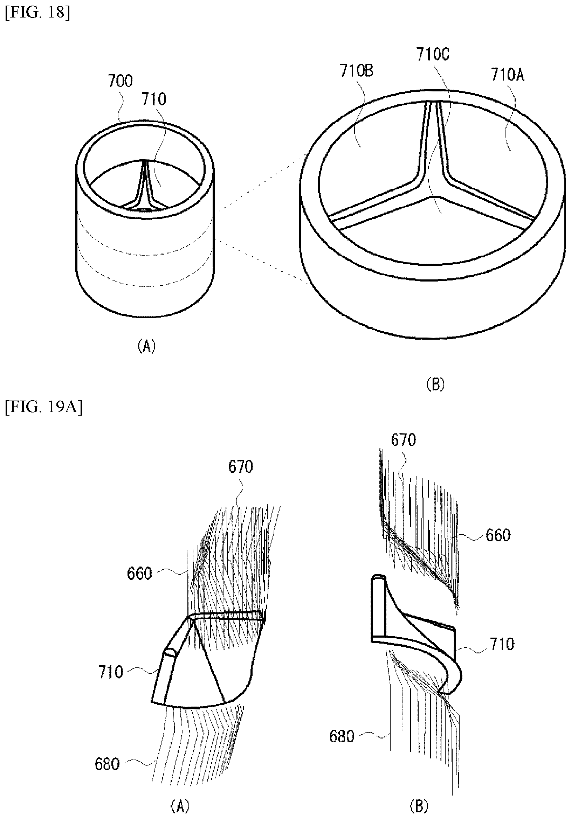

FIG. 18 is a diagram exemplifying a cell structure of a heart valve according to the present example. A tubular cell structure 700 is provided with a plurality of a valve part 710 extending from a tubular portion thereof to the inside (FIG. 18 (A)). As seen enlarged in FIG. 18 (B), the valve part 710 is configured from three valves 710A, 710B, and 710C.

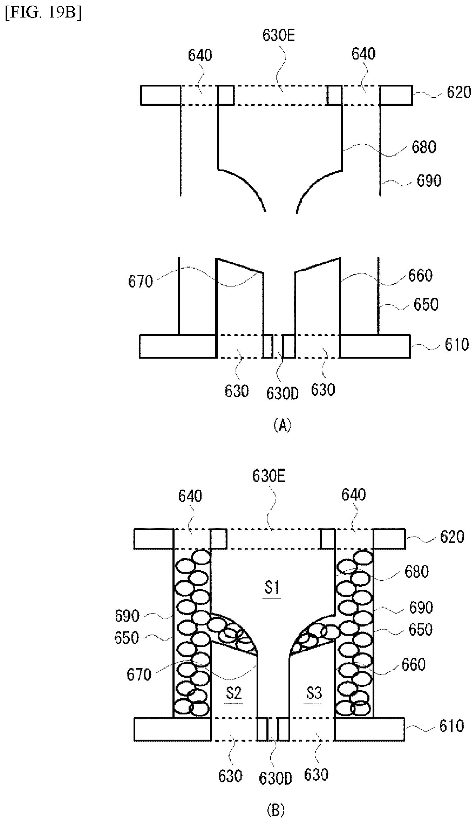

FIG. 19A is a diagram describing molding a cell structure according to the present example. The contour of the valve part 710 is defined by a space between the valve-molded needle-shaped member lower part 680, which has a convex shape, and the valve-molded needle-shaped member middle part 670, which is spread out in a fan-shape. FIG. 19B is a schematic cross-sectional diagram for describing molding a cell structure according to the present example. FIG. 19B (A) illustrates a state wherein the upper frame 610 and the lower frame 620 of the production device 100A are separated. The production device 100A, as illustrated in FIG. 16, is formed by combining the upper frame 610 and the lower frame 620. Although not illustrated, the positions of and space between the upper frame 610 and the lower frame 620 are adjusted by a pin 120, as illustrated in FIG. 1.

FIG. 19B(B) illustrates a state wherein a cell aggregate 400 is input from the input hole 640. The cell aggregate 400 accumulates at a thick portion of the tube of the cell structure 700 defined by the first outer needle-shaped member 650 and the second outer needle-shaped member 690, and when a certain amount has accumulated, it accumulates in a space where the valve part 710 defined by the valve-molded needle-shaped member lower part 680 and the valve-molded needle-shaped member middle part 670 is formed. A space S1 is a space having a culture medium circulation hole 630E at one end thereof, and a culture medium circulation hole 630D at another end opposing that end, and a culture solution 210 may be circulated thereby. Furthermore, a space S2 and a space S3 communicate with the culture medium circulation hole 630 (A, B, or C), and the culture solution 210 may be supplied to these spaces also. Note that the input hole 640 for inputting the cell aggregate 400 is provided to the lower frame 620; therefore, in FIG. 19B, the lower frame 620 is described at the top of the drawing.

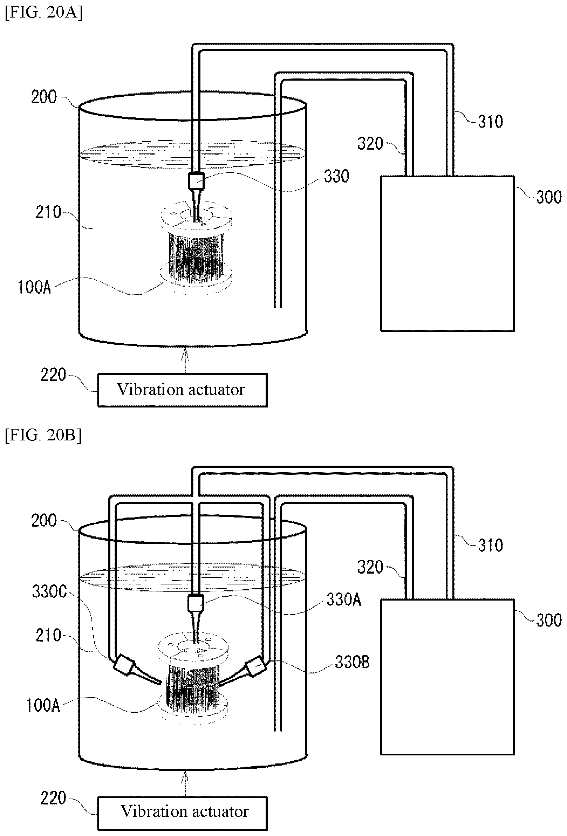

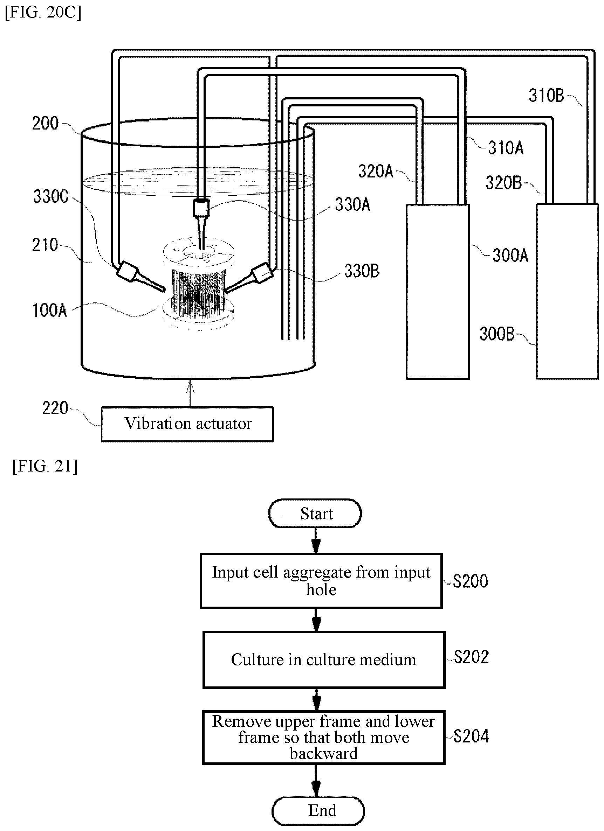

FIG. 20A is a diagram illustrating a culturing example for a cell aggregate according to the present example. The production device 100A is immersed in the culture solution 210 as in the production system illustrated in FIG. 14A. A culture solution supplied from a circulation pump 300 is discharged from a nozzle 300; thus, the culture solution may be circulated from the culture medium circulation holes 630E to 630D. FIG. 20B is a diagram illustrating a culturing example using a plurality of a nozzle according to the present invention. In this case, a nozzle 330B and a nozzle 330C for supplying a culture medium from the periphery of the cell aggregate 400 are used in addition to a nozzle 330A inserted into the culture medium circulation hole 630E. Furthermore, similar to the production system illustrated in FIG. 14B, each nozzle may be fixed at an arbitrary position. FIG. 20C is a diagram illustrating a culturing example according to the present invention wherein there is a plurality of a nozzle and a culture medium of another system is supplied simultaneously. In the production system illustrated in FIG. 20, culturing using the circulation pumps 300A and 300B from another system is possible, similar to the production system illustrated in FIG. 14C. Nozzles 330B and 330C illustrated in FIGS. 20B and 20C are preferably fixed close to the culture medium circulation hole 630 and supply the culture solution 210 to the spaces S2 and S3.

FIG. 21 is a flowchart showing a culturing example according to the present invention wherein there is a plurality of a nozzle and a culture medium of another system is supplied simultaneously. First, the cell aggregate 400 is input from the input hole 640 of the production device 100A (S200). Following, as illustrated in FIGS. 20A to 20C, the cell aggregate is cultured in a culture medium (S202), and the upper frame 610 and the lower frame 620 are removed to separate from the cell structure 700.

Preferred embodiments of the present invention have been described in detail above; however, the present invention is not limited to specific embodiments. Various modifications and changes are possible within the scope of the summary of the invention described in the Scope of Patent Claims.

**Description of Reference Numerals**

| | |
|---|---|
| 110, 110A: production device | 110: top plate |
| 112: through hole | 114: input hole |
| 116: culture medium circulation hole | 118: needle hole |
| 120: pin | 122: male screw part |
| 124: male screw part | 126: ring member |
| 130: first slide plate | 132: through hole |
| 134: needle hole | 135: culture medium circulation hole |
| 145: culture medium circulation hole | 140: second slide plate |
| 142: through hole | 144: needle hole |
| 150: stopper | 152: grip part |
| 154: culture medium circulation hole | 156: slide guide |
| 160: base plate | 162: female screw part |
| 164: through hole | 170: outer circumference needle-shaped member |
| 172: needle-shaped member | 180: inner circumference needle-shaped member |
| | |
| 182: needle-shaped member | 200: container |
| 210: culture solution | 220: vibration actuator |
| 300: circulation pump | 310: tube |
| 320: tube | 330: nozzle |
| 400: cell aggregate | 500: cell structure |
| 610: upper frame | 620: lower frame |
| 630: culture medium circulation hole | 640: input hole |
| 650: first outer needle-shaped member | 660: valve-molded needle-shaped member upper part |
| 670: valve-molded needle-shaped member middle part | |
| 680: valve-molded needle-shaped member lower part 680 | |
| 690: second outer needle-shaped member | 700: cell structure |
| 710: valve part | |

## Claims

1. A cell structure production device, the production device comprising:
at least one upper member,
at least one lower member, and
a plurality of linear members disposed between the upper member and the lower member, wherein one end of each of the linear members is supported by the upper member, and the other end is supported by the lower member, and
the plurality of linear members define a three-dimensional space, and the space is capable of accommodating a plurality of cell aggregates.

2. The production device according to claim 1, wherein the plurality of linear members comprises a first linear member substantially defining a first surface, and a second linear member substantially defining a second surface separated from the first surface, and the production device accommodates a cell aggregate in a space formed by the first surface and the second surface.

3. The production device according to claim 2, wherein the first surface defines an outer shape of the cell structure, and the second surface defines the inner shape of the cell structure.

4. The production device according to claim 2 or 3, wherein the first surface and the second surface are a spherical surface or a curved surface.

5. The production device according to any one of claims 1 to 4, wherein a cell aggregate accommodated within the space is exposed to a liquid such as a culture medium through the plurality of linear members.

6. The production device according to any one of claims 1 to 5, wherein the upper member comprises a substantially flat member, and an input hole for inputting the cell aggregate within the space is formed on the flat member.

7. The production device according to any one of claims 1 to 6, wherein the production device further comprises a slide member having a plurality of a through hole formed between the upper member and the lower member, the plurality of linear members penetrating the plurality of a through hole, wherein the slide member is capable of moving between the upper member and the lower member.

8. The production device according to any one of claims 1 to 7, wherein the production device further comprises a plurality of a strut for connecting the upper member and the lower member, wherein the slide member is guided by the plurality of a strut and is capable of moving in a direction close to or separated from the upper member.

9. The production device according to claim 8, wherein at least one of the plurality of a strut is provided with a control member for controlling movement of the slide member.

10. The production device according to claim 2 or 3, wherein the first and second linear members define a structure of blood vessels.

11. The production device according to claim 2 or 3, wherein the first and second linear members define a structure of a valve of a heart.

12. The production device according to any one of claims 1 to 11, wherein the plurality of linear members is a member produced by a three-dimensional printer.

13. The production device according to any one of claims 1 to 12, wherein the plurality of linear members is a member produced by a wire-bonding method, such as that used in semi-conductor production.

14. The production device according to any one of claims 1 to 13, wherein the plurality of linear members is a member produced by formation using a mold.

15. A production system for producing a cell structure, comprising the production device according to any one of claims 1 to 14, a container capable of accommodating the production device and for accommodating a culture medium for providing nourishment to a cell aggregate in the production device, and a pump for circulating the culture medium.

16. The production system according to claim 15, wherein the production system further comprises shaking means for shaking the container.

17. The production system according to claim 15 or 16, wherein the production system further comprises means for arbitrarily supplying a culture medium to a defined site.

18. A production method for a cell structure utilizing the production device according to any one of claims 1 to 14, wherein the production method comprises:
a step of supplying a plurality of cell aggregates to the space,
a step of culturing the cell aggregates and fusing the cell aggregates, and
a step of disengaging the plurality of linear members from the fused cell aggregates.

19. The production method according to claim 18, wherein the slide member is moved to disengage the plurality of linear members from cell aggregates.

20. The production method according to claim 18 or 19, wherein the step of disengaging the plurality of linear members comprises a step of separately disengaging each linear member.
